# EUROPEAN PATENT SPECIFICATION

(11) **EP 3 198 298 B1**
(45) Date of publication and mention of the grant of the patent: **16.10.2019**
(21) Application number: 14809109.3
(22) Date of filing: 24.09.2014
(51) Int. Cl.: G01S 7/52, A61B 8/08, A61B 8/00

(54) **TRANSDUCER ORIENTATION MARKER**
ORIENTIERUNGSMARKER EINES WANDLERS
MARQUEUR D'ORIENTATION DE TRANSDUCTEUR

(43) Date of publication of application: 02.08.2017
(73) Proprietor: B-K Medical ApS, 2730 Herlev (DK)
(72) Inventor: ANTOL, John J., Nahant, MA 01908 (US); SASADY, Niels-Christian, DK-2000 Frederiksberg (DK); JENSEN, Henrik, DK 2880 Bagsvaerd (DK)
(74) Representative: Gevers Patents
(86) International application number: PCT/IB2014/002532
(87) International publication number: WO 2016/046588

(56) References cited:
- JP-A- H09 192 128
- US-A1- 2006 176 242
- US-A1- 2010 010 348
- US-A1- 2013 211 243

## Description

### TECHNICAL FIELD

The following generally relates to ultrasound imaging and more particularly to an ultrasound imaging transducer orientation marker.

### BACKGROUND

An ultrasound imaging system has included an ultrasound probe and a console. The console includes a processor and memory, application software, a communication interface connector, etc. and interfaces with a display monitor and a user interface. The ultrasound probe includes a housing, a transducer array housed by the housing, and cable with connector. The probe and console communicate through the connectors.

The transducer array includes transducing elements that transmit an ultrasound signal in response to being excited and that sense echoes produced in response to the signal interacting with structure. In B-mode, the echoes are processed, producing a sequence of focused, coherent echo samples along focused scanlines of a scanplane. The scanlines are scan converted into a format of a display monitor and visually presented as image via the display monitor.

The probe housing has included a small fin near one side of the transducer array that protrudes out from the housing. The fin indicates a left/right orientation of the transducer array. By convention, the fin should point toward the patient's right side in transverse views and head in longitudinal views. The displayed image has been overlaid with an on-screen marking that corresponds to the fin. The side of the image corresponding with the fin end of the transducer is shown onscreen with a colored orientation marker. In this manner, the sonographer will be visually apprised of the image plane and orientation of the displayed image.

To add an orientation fin to a probe that does not already have an orientation fin, the mold tooling would need to be changed to include the orientation fin, which would add cost. For probes with two part handles, the orientation fin should be at the dividing line, which requires extra care. For probes that already have an orientation fin, the fin may make it more difficult to clean the probe as it may require extra time and/or additional cleaning attention around and at the fin. US2010010348 discloses the state of the art.

### SUMMARY

Aspects of the application address the above matters, and others.

In one aspect, an imaging system comprises an ultrasounds probe including a housing with a probe orientation marker disposed on the housing. The imaging system further comprises a display. The imaging system further comprises a console, electrically interfaced with the probe and the display, which includes a controller. The controller is configured to visually present an ultrasound image, in electronic format and via the display, with an image orientation marker visually displayed superimposed over the image and selectively located with respect to the displayed image based on the location of the probe orientation marker on the housing.

In another aspect, a method includes receiving a signal indicating an orientation of an ultrasound probe. The method further includes identifying, with a processor, the orientation of the ultrasound probe from the signal. The method further includes visually displaying, via a display, an ultrasound image generated with data detected by an array of transducer elements of the ultrasound probe. The method further includes overlaying, with the processor, a graphic over the displayed image, wherein the graphic mimics a graphic on the probe and indicates an image plane and orientation of the displayed image with respect to an image plane and orientation of the ultrasound probe.

In another aspect, an ultrasound probe including a transducer array with a plurality of transducer elements, a housing that supports the transducer array, wherein the housing includes an outer surface and a probe orientation marker embedded in the outer surface. The probe orientation marker identifies an imaging plane and a spatial orientation of the transducer array within the ultrasounds probe.

Those skilled in the art will recognize still other aspects of the present application upon reading and understanding the attached description.

### BRIEF DESCRIPTION OF THE DRAWINGS

The application is illustrated by way of example and not limitation in the figures of the accompanying drawings, in which like references indicate similar elements and in which:
Figure 1 schematically illustrates an example ultrasound imaging system;
Figure 2 illustrates a first side of an example probe;
Figure 3 illustrates a second side of the example probe of Figure 2 with a probe orientation marker;
Figure 4 illustrates the second side of the example probe of Figure 3 in connection with sub-windows displaying an image and an image orientation marker for different combinations of displayed image plane and orientation;
Figure 5 illustrates a variation of Figure 4 in which the probe orientation marker and the mage orientation marker include a graphic of a company logo;
Figure 6 illustrates a first side of another example probe;
Figure 7 illustrates a second side of the other example probe of Figure 6 with the probe orientation marker disposed thereon;
Figure 8 illustrates a first side of another example probe;
Figure 9 illustrates a second side of the other example probe of Figure 8 with the probe orientation marker disposed thereon;
Figure 10 illustrates a first side of another example probe;
Figure 11 illustrates a second side of the other example probe of Figure 10 with the probe orientation marker disposed thereon;
Figure 12 illustrates an example of the ultrasound imaging system; and

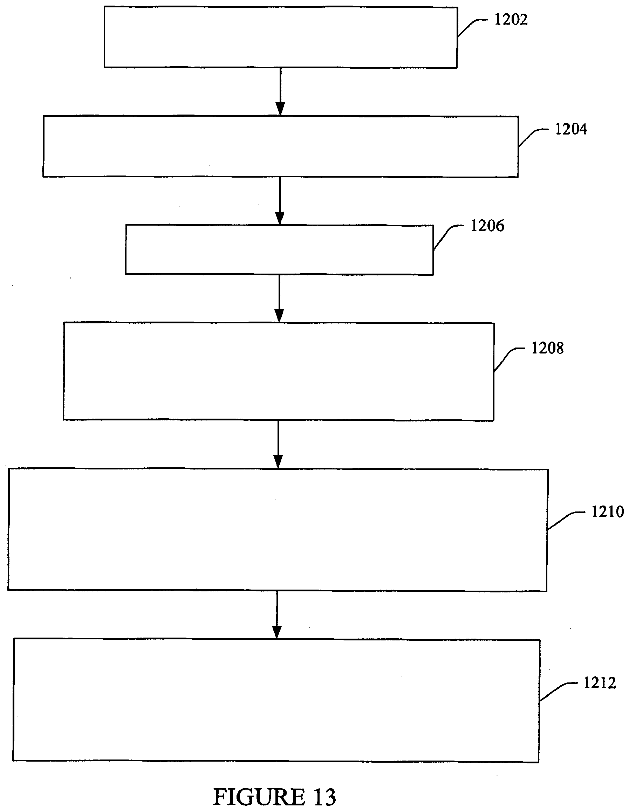

Figure 13 illustrates an example method in accordance with the description herein.

### DETAILED DESCRIPTION

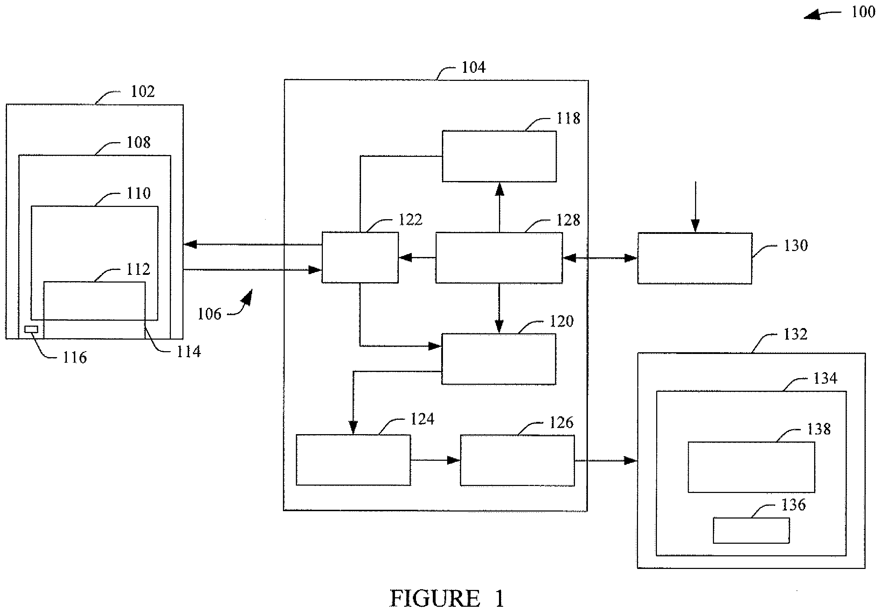

Figure 1 illustrates an example imaging system 100, such as an ultrasound imaging system.

The imaging system 100 includes a probe 102 and a console 104, which are configured to interface over a communications path 106. In one instance, the communications path 106 is through an electrical-mechanical connection of complementary connectors of the probe 102 and the console 104. In another instance, the communications path 106 is through respective wireless interfaces.

The probe 102 includes a housing 108, a transducer array 110 of transducer elements 112, and an acoustic window 114. The transducer array 110 can include one or more rows of the transducer elements 112, which are configured to transmit ultrasound signals and receive echo signals. Suitable arrays 110 include linear, curved, and/or otherwise shaped. The transducer array 110 can be fully populated or sparse.

The housing 108 houses or encloses the transducer array 110, which is mechanically supported by and/or within the housing 108. The acoustic window 114 is disposed in and/or about a material free region on a side of the housing 108 next to a transducing side of the transducer elements 112 and serves as an interface between the transducer array 110 and the surrounding environment.

The illustrated housing 108 has a probe orientation marker 116. As described in greater detail below, the probe orientation marker 116 is disposed at a predetermined location on the housing 108 and visually indicates information such as the image plane and/or an orientation of the probe 102. For example, in one instance, the probe orientation marker 116, with respect to the probe 102, indicates whether the probe 102 is facing up or down, left or right, etc.

The console 104 includes transmit circuitry 118 configured to generate a set of radio frequency (RF) pulses that are conveyed to the transducer array 110 and selectively excite a set of the transducer elements 112, causing the elements to transmit ultrasound signals. The console 104 further includes receive circuitry 120 that senses or receives echoes (RF signals) generated in response to the transmitted ultrasound signals interacting with structure (e.g., organ cells, blood cells, etc.).

The console 104 further includes a switch 122. The switch 122 switches between the transmit circuitry 118 and the receive circuitry 120, depending on whether the transducer array 110 is being operated in transmit mode or receive mode. In transmit mode, the switch 122 electrically connects the transmit circuitry 118 to the transducer elements 112. In receive mode, the switch 122 electrically connects the receive circuitry 120 to the transducer elements 112.

The console 104 further includes an echo processor 124 that processes received echoes. Such processing may include applying time delays, weighting on the channels, summing, and/or otherwise beamforming received echoes. In B-mode, the echo processor 124 produces a sequence of focused, coherent echo samples along focused scanlines of a scanplane. Other processing may lower speckle, improve specular reflector delineation, and/or includes FIR filtering, IIR filtering, etc.

The console 104 further includes a scan converter 126. The scan converter 126 scan converts the output of the echo processor 124 creating images for display. The console 104 further includes a controller 128. The controller 128 controls one or more of the transmit circuitry 118, the receive circuitry 118, the switch 122, the echo processor 124 and/or the scan converter 126. Such control can be based on available modes of operation. Examples of such modes of operation include one or more of B-mode, Doppler mode, etc.

In the illustrated embodiment, the console 104 also interfaces a user interface (UI) 130 and a display 132. In another embodiment, at least one of the user interface 130 or the display 132 are integrated in and part of the console 104. The UI 130 may include one or more input devices (e.g., buttons, knobs, trackball, etc.) and/or one or more output devices (e.g., visual, audio, etc. indicators). The UI 130 can be used to select an imaging mode, etc.

The display 132 includes an image window 134 configured to display an image, such as an ultrasound image 136, and an image orientation marker 138, which indicates an orientation of the displayed image. As described in greater detail below, in one non-limiting instance, the image orientation marker 138 includes a graphic, which mirrors the probe orientation marker 116 in that the graphic visually resembles or looks the same as the probe orientation marker 116. The image orientation marker 138 is overlaid over the image based on a predetermined location so at to indicate an orientation of the displayed image with respect to the current actual physical orientation of the probe 102 with respect to the subject or object being scanned.

It is to be appreciated that the console 104 includes one or more processor (e.g., a central processing unit, a microprocessor, etc., and memory or computer readable medium which excludes transitory and includes physical memory) encoded with computer executable instructions. The instructions, when executed by the processor, cause the processor to perform one or more of the functions described herein.

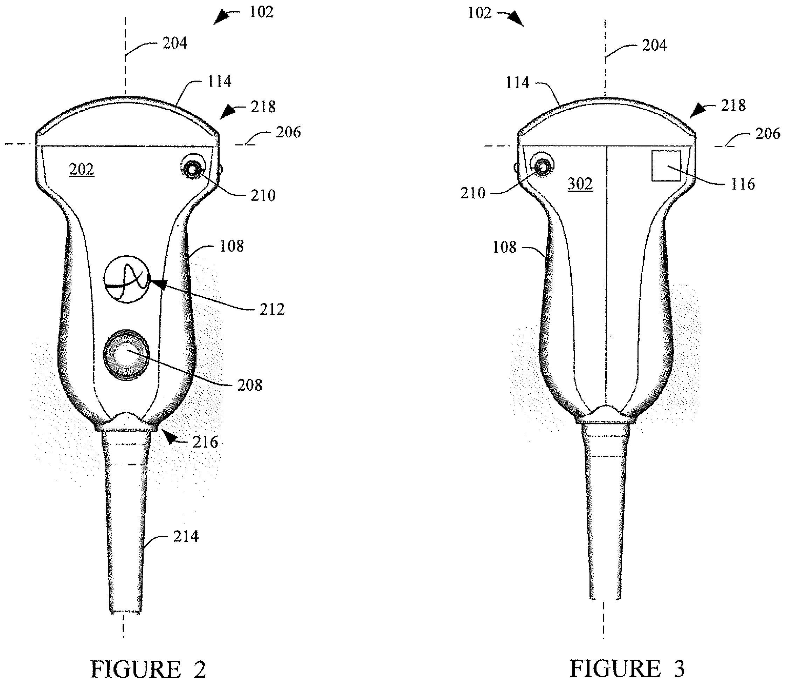

Figures 2 and 3 depict an example of the probe 102. Figure 2 shows a top side 202 of the probe 102, and Figure 3 shows a bottom side 302 of the probe 102. The top side 202 of the probe 102 and the bottom side 302 of the probe 102 are opposing sides of the probe 102.

With reference to Figures 2 and 3, the probe 102 has a long axis 204 and a short axis 206, which is transverse to the long axis 204. The transducer elements 112, which are not visible in Figures 2 and 3 since they are located within the housing 108 and behind the acoustic window 114, extend sequentially along the short axis 206 in the azimuth direction.

The illustrated top side 202 (Figure 2) includes at least one user control 208, which is integrated in the top side 202. For example, the user control 208 can be a physical button extending from inside of the housing 108, through an opening in the housing 108, and protruding out from the housing 108. In another instance, the user control 208 can be part of a touch sensitive surface of the housing 108. The illustrated housing 108 further includes a puncture guide 210.

The illustrated housing 108 further includes a graphic 212 (Figure 2 only). In the illustrated example, the graphic 212 includes a logo. The probe 102 also includes a cable 214 which routes electrical channels between the individual transducer elements 112 and the connector of the cable. The cable 214 extends from a back 216 of the probe 102, which is opposite a probe head 218, which includes the acoustic window 114 and the transducer elements 112.

The bottom side 302 (Figure 3) includes the probe orientation marker 116. The probe orientation marker 116 is disposed in an orientation in which it is non-inverted when the probe is in scanning mode, e.g., meaning the array 110 pointing down at the object and the cable 214 pointing up away from the object. The probe orientation marker 116 is disposed proximate to the probe head 218 and outer peripheral transducer elements of the transducer array 110, symmetrically disposed with respect to puncture guide 210 about the long axis 204, and aligned with respect to puncture guide 210 about the long axis 204.

The illustrated probe orientation marker 116 occupies a square footprint. A size of the illustrated probe orientation marker 116 is a size on an order of 2.5 to 7.5 ± 0.05 millimeters (mm), such as 4.8 ± 0.05 mm, 4.9 ± 0.05 mm, 5.0 ± 0.05 mm, 5.1 ± 0.05 mm, or 5.2 ± 0.05 mm, or other size in the range of 2.5 to 7.5 ± 0.05 mm. In another embodiment, the probe orientation marker 116 can be smaller or larger and/or occupy a rectangular, circular, oval, irregular, etc. footprint.

In one instance, the probe orientation marker 116 is an engravement in the housing 108. For example, the probe orientation marker 116 can be laser and/or otherwise engraved in the housing 108. Generally, an engravement is easier to clean relative to a protrusion type (e.g., a fin) physical marker. In another instance, the probe orientation marker 116 can be tattooed, painted on, and/or otherwise placed on the housing 108. The probe orientation marker 116 may or may not include color.

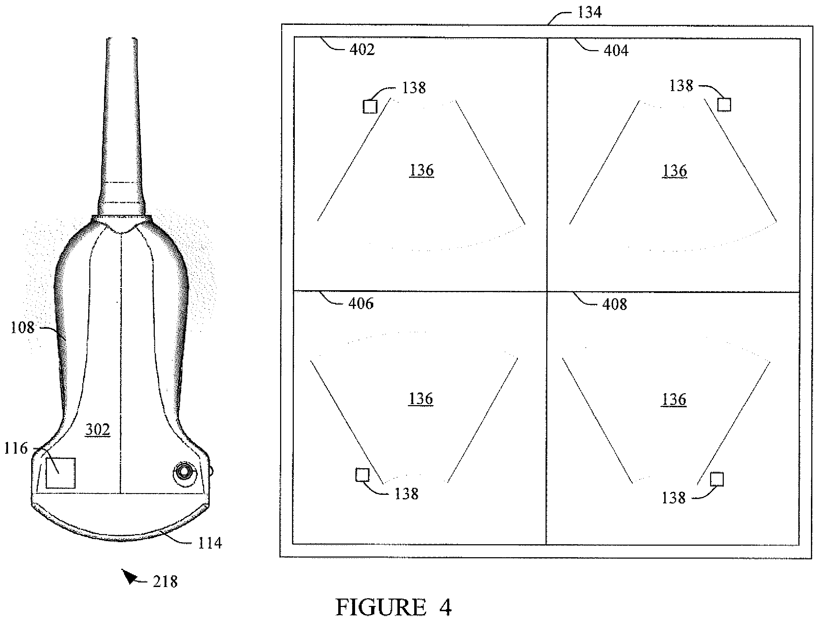

Figure 4 depicts the probe 102 of Figures 2 and 3 in connection with the image window 134 of the display 132. In this example, a plurality of sub-windows 402, 404, 406 and 408 are displayed in the image window 134, each with a different image plane and orientation.

The sub-window 402 shows an image plane and orientation corresponding to the orientation of the illustrated probe 102. That is, the probe head 218 faces down and the probe orientation marker is on the left side of the probe. As such, the image plane is shown facing down and the image orientation marker 138 is on the upper left side of the displayed image.

In the sub-window 404, the orientation has been rotated 180 degrees (e.g., from left to right), and the image orientation marker 138 is on the upper right side of the displayed image (e.g., moved from left to right). The image plane remains facing down. Alternatively, this represents the orientation of the probe 102 after rotating the probe 102 180 degrees while keeping the probe head 218 facing down.

In the sub-window 406, the image plane has been rotated 180 degrees (e.g., from down to up), and the image orientation marker 138 is on the lower right side of the displayed image. The image plane faces up. Alternatively, this represents the orientation of the probe 102 after rotating the probe 102 180 degrees to face up while keeping the orientation the same.

In the sub-window 408, both the image plane and the orientation have been rotated 180 degrees. In this example, the image orientation marker 138 is on the lower right side of the displayed image and the image plane faces up. Alternatively, this represents the orientation of the probe 102 after rotating the probe 102 180 degrees to face up and rotating the probe head 218.

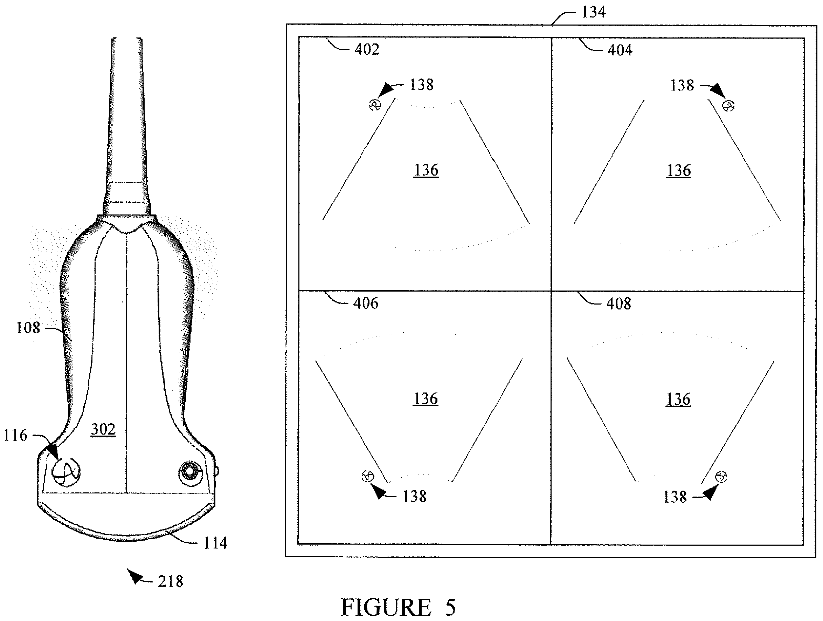

Figure 5 depicts the example described in connection with Figure 4 in which both the probe orientation marker 116 and the image orientation marker 138 are the same graphic. In this example, the graphic is a log such as a company logo. In another embodiment, at least one of the probe orientation marker 116 or the image orientation marker 138 is a different graphic.

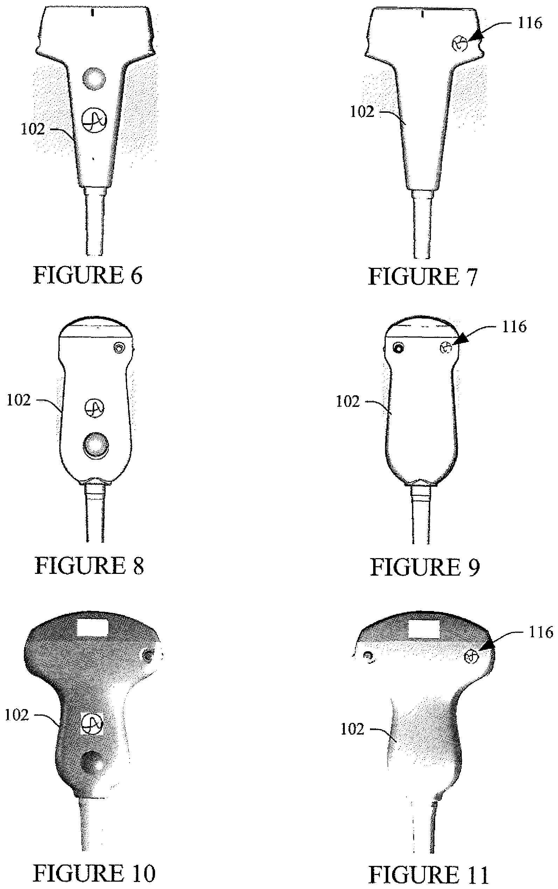

Figures 6, 7, 8, 9, 10 and 11 depict example of other probes 102 with the probe orientation markers 116. Figures 6 and 7 depict top and bottom views of the same probe. Figures 8 and 9 depict top and bottom views of the same probe. Figures 10 and 11 depict top and bottom views of the same probe. In a variation, the probe orientation marker 116 can additionally or alternatively be disposed on the top side 202. In another variation, different colors can be used to indicate the image plane and orientation instead of mirroring and/or inverting the probe orientation marker 116.

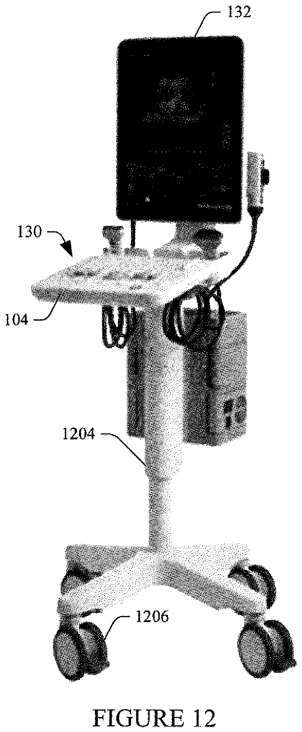

Figure 12 illustrates a non-limiting example of the ultrasound imaging system 100. In this example, the console 104 is affixed to a mobile cart 1204, which include movers 1206 such as wheels, casters, etc., the user interface 130 is part of console 104, and the display 132 is affixed to the mobile cart 1204. In another configuration, the ultrasound imaging system 100 does not include movers, but instead is configured to rest on a table, desk, etc.

Figure 12 illustrates a method.

It is to be appreciated that the order of the following acts is provided for explanatory purposes and is not limiting. As such, one or more of the following acts may occur in a different order. Furthermore, one or more of the following acts may be omitted and/or one or more additional acts may be added.

At 1202, the probe 102 is connected to the console 104.

At 1204, the probe head 218 of the probe is placed in acoustic communication with a subject or object to be scanned.

At 1206, the subject or object is scanned.

At 1208, an image generated in response to the scan is displayed with the image orientation marker 138 superimposed thereover to mirror the image plane and orientation of the actual probe 102.

At 1210, the location of the displayed image orientation marker 138 is changed in response to switching the display direction and/or orientation. In a variation, the act is omitted.

At 1212, the location of the image orientation marker 138 is changed in response to changing the physical direction and/or orientation of the probe. In a variation, the act is omitted.

At least a portion of the method discussed herein may be implemented by way of computer readable instructions, encoded or embedded on computer readable storage medium (which excludes transitory medium), which, when executed by a computer processor(s), causes the processor(s) to carry out the described acts. Additionally or alternatively, at least one of the computer readable instructions is carried by a signal, carrier wave or other transitory medium.

The application has been described with reference to various embodiments. Modifications and alterations will occur to others upon reading the application. It is intended that the invention be construed as including all such modifications and alterations, including insofar as they come within the scope of the appended claims and the equivalents thereof.

## Claims

1. An ultrasound imaging system, comprising:
an ultrasound probe (102) including a housing (108) with a probe orientation marker (116) disposed on the housing;
a display (132); and
a console (104), electrically interfaced with the probe and the display, that includes a controller (128) configured to visually present an ultrasound image, in electronic format and via the display, with an image orientation marker (138) visually displayed superimposed over the image and selectively located with respect to the displayed image at one of: an upper left side of the displayed image, an upper right side of the displayed image, a lower right side of the displayed image and a lower left side of the displayed image based on the location of the probe orientation marker (116) on the housing (108), and the image orientation marker (138) is a graphic of only the probe orientation marker (116) and is configured to mirror the probe orientation marker (116) in that the graphic visually resembles or looks the same as the probe orientation marker (116).

2. The imaging system of claim 1, wherein the probe orientation marker (116) is configured to identify a spatial orientation of the ultrasounds probe (102) with respect to a subject or object to scan.

3. The imaging system of claims 1 or 2, wherein the probe orientation marker (116) and the image orientation marker (138) are a same graphic.

4. The imaging system of claim 3, wherein the same graphic is a company logo.

5. The imaging system of any of claims 1 to 4, wherein the controller (128) is configured to visually present the image orientation marker (138) based on an image plane of the ultrasound probe (102).

6. The imaging system of claim 5, wherein the controller is configured to visually present the image orientation marker (138) to mirror the image plane of the ultrasound probe (102) with respect to a subject or object to scan.

7. The imaging system of any of claims 1 to 6, wherein the ultrasounds probe (102) includes a transducer array (110) of elements (112), and the probe orientation marker (116) is disposed on an outside of the housing (108) proximate to a region of the probe (102) housing an end region of the transducer array.

8. The imaging system of any of claims 1 to 7, wherein the housing (108) includes a long axis (204) and the probe orientation marker (116) is disposed on one side of the long axis.

9. The imaging system of any of claims 1 to 8, wherein the probe orientation marker (116) is disposed within a region having a size on an order of five millimeters.

10. The imaging system of any of claims 1 to 9, wherein the probe orientation marker (116) is an engravement in the housing (108).

11. A method of displaying orientation of a displayed image with respect to an ultrasound probe in an ultrasound imaging system, the method comprising the steps of:
receiving a signal indicating an orientation of an ultrasound probe (102);
identifying, with a processor, the orientation of the ultrasound probe (102) from the signal;
visually displaying, via a display, an ultrasound image generated with data detected by an array (110) of transducer elements (112) of the ultrasound probe (102); and
overlaying, with the processor, a graphic over the displayed image at one of: an upper left side of the display image, an upper right side of the display image, a lower right side of the display image and a lower left side of the display image, wherein the graphic mimics only a graphic on the ultrasound probe (102) and the location of the graphic with respect to the displayed image indicates an image plane and orientation of the displayed image with respect to an image plane and orientation of the ultrasound probe (102).

12. The method of claim 11, further comprising:
moving the location of the location of the graphic on the displayed image in response to a change in an orientation of the ultrasound probe (102).

13. The method of claim 11, further comprising:
moving the location of the location of the graphic on the displayed image from left to right or right to left in response to the rotating the ultrasound probe (102) from left to right or right to left; and/or
moving the location of the location of the graphic on the displayed image from up to down or down to up in response to the rotating the ultrasound probe (102) from up to down or down to up.

14. The method of claim 11, further comprising:
changing the location of the displayed image orientation marker (138) in response to switching the display direction and/or orientation.

15. The method of claim 11, further comprising:
changing the location of the displayed image orientation marker (138) in response to changing the physical direction and/or orientation of the ultrasound probe (102).

## Patentansprüche

1. Ultraschallbildgebungssystem, umfassend:
eine Ultraschallsonde (102), die ein Gehäuse (108) mit einer auf dem Gehäuse eingerichteten Sondenorientierungsmarkierung (116) beinhaltet;
eine Anzeige (132); und
eine Konsole (104), elektrisch an die Sonde und die Anzeige angeschlossen, die eine Steuereinheit (128) beinhaltet, die konfiguriert ist, um ein Ultraschallbild in elektronischem Format und mittels der Anzeige visuell darzustellen, mit einer Bildorientierungsmarkierung (138), die über dem Bild überlagert visuell angezeigt wird und sich in Bezug auf das angezeigte Bild selektiv an einer von einer oberen linken Seite des angezeigten Bildes, einer oberen rechten Seite des angezeigten Bildes, einer unteren rechten Seite des angezeigten Bildes und einer unteren linken Seite des angezeigten Bildes befindet, basierend auf der Stelle der Sondenorientierungsmarkierung (116) an dem Gehäuse (108), und die Bildorientierungsmarkierung (138) eine Grafik nur der Sondenorientierungsmarkierung (116) ist und konfiguriert ist, um die Sondenorientierungsmarkierung (116) so zu spiegeln, dass die Grafik der Sondenorientierungsmarkierung (116) visuell ähnelt oder wie sie aussieht.

2. Bildgebungssystem nach Anspruch 1, wobei die Sondenorientierungsmarkierung (116) konfiguriert ist, um eine räumliche Orientierung der Ultraschallsonde (102) in Bezug auf ein zu scannendes Subjekt oder Objekt zu identifizieren.

3. Bildgebungssystem nach Anspruch 1 oder 2, wobei die Sondenorientierungsmarkierung (116) und die Bildorientierungsmarkierung (138) die gleiche Grafik sind.

4. Bildgebungssystem nach Anspruch 3, wobei die gleiche Grafik ein Firmenlogo ist.

5. Bildgebungssystem nach einem der Ansprüche 1 bis 4, wobei die Steuereinheit (128) konfiguriert ist, um die Bildorientierungsmarkierung (138) basierend auf einer Bildebene der Ultraschallsonde (102) visuell darzustellen.

6. Bildgebungssystem nach Anspruch 5, wobei die Steuereinheit konfiguriert ist, um die Bildorientierungsmarkierung (138) visuell darzustellen, um die Bildebene der Ultraschallsonde (102) in Bezug auf ein zu scannendes Subjekt oder Objekt zu spiegeln.

7. Bildgebungssystem nach einem der Ansprüche 1 bis 6, wobei die Ultraschallsonde (102) ein Transducer-Array (110) von Elementen (112) beinhaltet und die Sondenorientierungsmarkierung (116) an einer Außenseite des Gehäuses (108) nahe einem Bereich der Sonde (102), der einen Endbereich des Transducer-Arrays beherbergt, eingerichtet ist.

8. Bildgebungssystem nach einem der Ansprüche 1 bis 7, wobei das Gehäuse (108) eine lange Achse (204) beinhaltet und die Sondenorientierungsmarkierung (116) an einer Seite der langen Achse eingerichtet ist.

9. Bildgebungssystem nach einem der Ansprüche 1 bis 8, wobei die Sondenorientierungsmarkierung (116) innerhalb eines Bereichs mit einer Größe in einer Größenordnung von fünf Millimetern eingerichtet ist.

10. Bildgebungssystem nach einem der Ansprüche 1 bis 9, wobei die Sondenorientierungsmarkierung (116) eine Eingravierung in dem Gehäuse (108) ist.

11. Verfahren zum Anzeigen einer Orientierung eines angezeigten Bildes in Bezug auf eine Ultraschallsonde in einem Ultraschallbildgebungssystem, wobei das Verfahren die folgenden Schritte umfasst:
Empfangen eines Signals, das eine Orientierung einer Ultraschallsonde (102) angibt;
Identifizieren der Orientierung der Ultraschallsonde (102) aus dem Signal mit Hilfe eines Prozessors;
visuelles Darstellen, mittels einer Anzeige, eines Ultraschallbildes, das mit durch ein Array (110) von Transducerelementen (112) der Ultraschallsonde (102) detektierten Daten erzeugt wird; und
Legen einer Grafik über das angezeigte Bild mit Hilfe des Prozessors an einer von einer oberen linken Seite des Anzeigebildes, einer oberen rechten Seite des Anzeigebildes, einer unteren rechten Seite des Anzeigebildes und einer unteren linken Seite des Anzeigebildes, wobei die Grafik nur eine Grafik auf der Ultraschallsonde (102) imitiert und die Stelle der Grafik in Bezug auf das angezeigte Bild eine Bildebene und -orientierung des angezeigten Bildes in Bezug auf eine Bildebene und -orientierung der Ultraschallsonde (102) angibt.

12. Verfahren nach Anspruch 11, weiter umfassend:
Bewegen der Stelle der Grafik auf dem angezeigten Bild in Reaktion auf eine Veränderung einer Orientierung der Ultraschallsonde (102).

13. Verfahren nach Anspruch 11, weiter umfassend:
Bewegen der Stelle der Grafik auf dem angezeigten Bild von links nach rechts oder rechts nach links in Reaktion auf das Drehen der Ultraschallsonde (102) von links nach rechts oder rechts nach links; und/oder
Bewegen der Stelle der Grafik auf dem angezeigten Bild von oben nach unten oder unten nach oben in Reaktion auf das Drehen der Ultraschallsonde (102) von oben nach unten oder unten nach oben.

14. Verfahren nach Anspruch 11, weiter umfassend:
Verändern der Stelle der angezeigten Bildorientierungsmarkierung (138) in Reaktion auf das Umschalten der Anzeigerichtung und/oder -orientierung.

15. Verfahren nach Anspruch 11, weiter umfassend:
Verändern der Stelle der angezeigten Bildorientierungsmarkierung (138) in Reaktion auf eine Veränderung der physikalischen Richtung und/oder Orientierung der Ultraschallsonde (102).

## Revendications

1. Système d'imagerie à ultrasons comprenant :
une sonde à ultrasons (102) incluant un boîtier (108) avec un marqueur d'orientation de sonde (116) disposé sur le boîtier ;
un affichage (132) ; et
une console (104) interfacée électriquement avec la sonde et l'affichage, qui inclut un dispositif de commande (128) configuré pour présenter visuellement une image à ultrasons, en format électronique et via l'affichage, avec un marqueur d'orientation d'image (138) visuellement affiché superposé à l'image et localisé sélectivement par rapport à l'image affichée sur l'un d'un côté gauche supérieur de l'image affichée, d'un côté droit supérieur de l'image affichée, d'un côté droit inférieur de l'image affichée et d'un côté gauche inférieur de l'image affichée sur la base de la localisation du marqueur d'orientation de sonde (116) sur le boîtier (108), et le marqueur d'orientation d'image (138) est un graphique du marqueur d'orientation de sonde (116) seulement et est configuré pour refléter le marqueur d'orientation de sonde (116) en ce sens que le graphique ressemble visuellement ou semble le même que le marqueur d'orientation de sonde (116).

2. Système d'imagerie selon la revendication 1, dans lequel le marqueur d'orientation de sonde (116) est configuré pour identifier une orientation spatiale de la sonde à ultrasons (102) par rapport à un sujet ou à un objet à balayer.

3. Système d'imagerie selon les revendications 1 ou 2, dans lequel le marqueur d'orientation de sonde (116) et le marqueur d'orientation d'image (138) sont un seul et même graphique.

4. Système d'imagerie selon la revendication 3, dans lequel le même graphique est un logo de société.

5. Système d'imagerie selon l'une quelconque des revendications 1 à 4, dans lequel le dispositif de commande (128) est configuré pour présenter visuellement le marqueur d'orientation d'image (138) sur la base d'un plan image de la sonde à ultrasons (102).

6. Système d'imagerie selon la revendication 5, dans lequel le dispositif de commande est configuré pour présenter visuellement le marqueur d'orientation d'image (138) afin de refléter le plan image de la sonde à ultrasons (102) par rapport à un sujet ou à objet à balayer.

7. Système d'imagerie selon l'une quelconque des revendications 1 à 6, dans lequel la sonde à ultrasons (102) inclut un réseau transducteur (110) d'éléments (112) et le marqueur d'orientation de sonde (116) est disposé à l'extérieur du boîtier (108) à proximité d'une région de la sonde (102) logeant une région d'extrémité du réseau transducteur.

8. Système d'imagerie selon l'une quelconque des revendications 1 à 7, dans lequel le boîtier (108) inclut un axe long (204) et le marqueur d'orientation de sonde (116) est disposé sur un côté de l'axe long.

9. Système d'imagerie selon l'une quelconque des revendications 1 à 8, dans lequel le marqueur d'orientation de sonde (116) est disposé au sein d'une région ayant une taille de l'ordre de cinq millimètres.

10. Système d'imagerie selon l'une quelconque des revendications 1 à 9, dans lequel le marqueur d'orientation de sonde (116) est une gravure dans le boîtier (108).

11. Procédé d'affichage d'orientation d'une image affichée par rapport à une sonde à ultrasons dans un système d'imagerie à ultrasons, le procédé comprenant les étapes consistant à :
recevoir un signal indiquant une orientation d'une sonde à ultrasons (102) ;
identifier, avec un processeur, l'orientation de la sonde à ultrasons (102) à partir du signal ;
afficher visuellement, via un appareil d'affichage, une image à ultrasons générée avec des données détectées par un réseau (110) d'éléments transducteurs (112) de la sonde à ultrasons (102) ; et
superposer, avec le processeur, un graphique sur l'image affichée sur l'un d'un côté gauche supérieur de l'image d'affichage, d'un côté droit supérieur de l'image d'affichage, d'un côté droit inférieur de l'image d'affichage et d'un côté gauche inférieur de l'image d'affichage, dans lequel le graphique ressemble à un graphique seulement sur la sonde à ultrasons (102) et la localisation du graphique par rapport à l'image affichée indique un plan image et une orientation de l'image affichée par rapport à un plan image et à une orientation de la sonde à ultrasons (102).

12. Procédé selon la revendication 11, comprenant en outre :
le déplacement de la localisation du graphique sur l'image affichée en réponse à un changement d'orientation de la sonde à ultrasons (102).

13. Procédé selon la revendication 11, comprenant en outre :
le déplacement de la localisation du graphique sur l'image affichée de la gauche à la droite ou de la droite à la gauche en réponse à la rotation de la sonde à ultrasons (102) de la gauche à la droite ou de la droite à la gauche ; et/ou
le déplacement de la localisation du graphique sur l'image affichée de haut en bas ou de bas en haut en réponse à la rotation de la sonde à ultrasons (102) de haut en bas ou de bas en haut.

14. Procédé selon la revendication 11, comprenant en outre :
le changement de la localisation du marqueur d'orientation d'image affiché (138) en réponse à la commutation de la direction et/ou de l'orientation d'affichage.

15. Procédé selon la revendication 11, comprenant en outre :
le changement de la localisation du marqueur d'orientation d'image affiché (138) en réponse au changement de la direction et/ou de l'orientation physique de la sonde à ultrasons (102).
